# EUROPEAN PATENT SPECIFICATION

(11) **EP 2 874 507 B1**
(45) Date of publication and mention of the grant of the patent: **30.12.2020**
(21) Application number: 13737612.5
(22) Date of filing: 18.07.2013
(51) Int. Cl.: A61K 31/07, A61K 47/14, A61K 47/22, A61K 47/26, A61K 47/36, A61K 31/035, A61K 31/11, A61K 31/232, A23K 20/179, A23L 29/10, A23L 5/44, A23L 33/105, A61K 9/107

(54) **AQUEOUS TRANSPARENT OIL-IN-WATER EMULSION COMPRISING AN EMULSIFIED CAROTENOID**
WÄSSRIGE TRANSPARENTE ÖL-IN-WASSER-EMULSION MIT EMULGIERTEM CAROTENOID
ÉMULSION HUILE DANS EAU TRANSPARENTE AQUEUSE COMPRENANT UN CAROTÉNOÏDE ÉMULSIFIÉ

(30) Priority: 20.07.2012 EP 12177364; 20.07.2012 US 201261673786 P
(43) Date of publication of application: 27.05.2015
(73) Proprietor: BASF SE, 67056 Ludwigshafen am Rhein (DE)
(72) Inventor: HELGASON, Thrandur, 67063 Ludwigshafen (DE); KÖPSEL, Christian, 69469 Weinheim (DE); SAMBALE, Clemens, 67459 Böhl-Iggelheim (DE); HABICH, Andreas, 67346 Speyer (DE); HASSE, Andreas, 67256 Weisenheim/Sand (DE)
(86) International application number: PCT/EP2013/065143
(87) International publication number: WO 2014/013002

(56) References cited:
- EP-A1- 0 966 889
- WO-A1-2012/059286
- DE-U1-202007 003 800
- JP-B2- 3 145 584
- US-A- 5 612 485
- US-A1- 2004 235 787
- US-A1- 2010 120 713
- US-A1- 2011 293 678

## Description

The invention relates to an aqueous transparent oil-in-water emulsion comprising a carotenoid and a process for producing said emulsion.

Emulsions containing carotenoids are well known in the state of the art. Carotenoids are used as colouring material and as active substances for the human food and animal feed industry as well as in the pharmaceutical sector. In this area they are also used in the last years to replace synthetic azo dyes, otherwise known as the 'Southampton Six': sunset yellow (E110), quinoline yellow (E104), carmoisine (E122), allura red (E129), tartrazine (E102), and Ponceau 4R (E124). These might have negative effects, especially an adverse effect on activity and attention in children.
In the past it was difficult to replace the azo dyes by carotenoids and to maintain exactly the same colour match of the products as with azo dyes.
Carotenoids are insoluble in water. Additionally, they show a great sensitivity to oxidation. Therefore, it is difficult to use the carotenoids in crystalline form which is not stable during storage. Generally, a lot of additives are necessary in order to obtain stable carotenoid emulsions, where the carotenoid is protected from oxidation and the emulsions have an improved colouration.

One approach to overcome the problems in handling carotenoids is the manufacturing of micro-emulsions. These micro-emulsions exhibit a soapy taste caused by the used emulsifiers.
Emulsifiers, especially ester of long-chain fatty acid with ascorbic acid, in particular ascorbyl palmitate, are also used according to the teaching of US 4,844,934 to obtain an emulsion stable to creaming.

Formation of small emulsions, made by dispersing a liquid oil such as Vitamin E acetate into water phase with a emulsifier followed by homogenization at high pressure, requires high amounts of energy with high amount of emulsifier (these are common equipment used for emulsion formation) because the oil phase is liquid at room temperature and the interfacial tension between Vitamin E acetate oil and the water phase is lower than regular vegetable oils (e.g. sunflower oil).
On the other hand, it is very much more difficult to emulsify carotenoids because of their low oil solubility and very low water solubility. Furthermore, preventing recrystallization of the carotenoids requires that the ratio of cis vs. trans isomers will be finely tuned to achieve a stable system. Therefore, emulsion containing carotenoids is in fact a different system than emulsion containing pure Vitamin E acetate. Finally, emulsion droplets containing only oil soluble vitamin (such as Vitamin E acetate) cannot be used as colorant.

As further prior art the following can be mentioned: WO 2012/059286 A1; EP 0 966 889 A1; US 2010/120713 A1; US 2011/293678 A1; US 2004/235787 A1; DE 20 2007 003800 U1; US 5,612,485 and JP 3 145584 B2.

It is object of the present invention to put aqueous carotenoid emulsions at disposal which show improved colour, preferably at high carotenoid concentrations, with improved bioavailability of the carotenoids. Additionally, the emulsion has to be clear and transparent, meaning a very low turbidity. A further object of the present invention is to use exclusively naturally renewable raw material or naturally identically raw materials. Furthermore, the emulsion has to be producible in an easy and effective way within only few steps.
It is further object of the present invention to put a carotenoid containing aqueous emulsions or a carotenoid containing powder as colorant at disposal which replace azo dyes and replicates the visual appearance of the end product coloured with azo dyes. Additionally, is object of the invention to put aqueous carotenoid emulsions at disposal which does not contain any azo dyes but replicates the visual appearance of a product coloured with azo dyes.

These problems are solved by the matters outlined in the claims.

The emulsion of the present invention is an aqueous transparent oil-in-water emulsion comprising an emulsified carotenoid in a concentration of 0.025-2000 ppm, preferably 0.025-300 ppm, 0.1-200 ppm, 0.1-100 ppm, 1-50 ppm, 1-30 ppm, 3 - 30 ppm, 3 - 20 ppm, more preferably 1-15 ppm, 3 - 15 ppm, especially 3-6 ppm. In one embodiment the concentration of the emulsified carotenoid is 5 ppm.
In one embodiment 1 ppm is 1 part per weight in 1 million parts per weight.
For the purpose of this invention an oil-in-water emulsion comprising an emulsified carotenoid means an emulsion of oil-droplets in water, whereby the carotenoid is present in the droplets and solved in the oil.

According to the present invention the term aqueous transparent oil-in-water emulsion means a clear emulsion with a low turbidity. The turbidity is measured with NTU which is nephelometric turbidity units that are measured with any standard turbidimeter, in one embodiment of the invention with HACH 2100AN Turbidimeter.
For the purpose of this invention a transparent emulsion is defined as an aqueous emulsion containing 5 ppm carotenoid with a NTU value below 35.
In the invention the aqueous emulsion containing 5 ppm has a turbidity value of 1 - 35 NTU, preferably 10 - 35 NTU, more preferably 15 - 30 NTU, especially 15 - 25 NTU, 15 - 20 NTU.
In a further embodiment of the invention the aqueous emulsion containing 5 ppm carotenoid has a turbidity value selected from the group consisting of: 15, 16, 17, 18, 19, 20, 21, 22, 23, 24 and 25 NTU.

In the invention the carotenoid is selected from the group consisting of: cantaxanthin, astaxanthin, lutein, zeaxanthin, beta-zeacaroten, lycopene, apocarotenal, bixin, paprika olioresin, capsanthin and capsorubin, preferably apocarotenal, more preferably beta-apo-8-carotenal.

In the context of this invention, the characteristic "free from" means that the relevant compounds cannot be detected in the composition of the invention with the known state-of-the-art analytical methods for qualitatively and/or quantitatively detecting carotenoids, for example spectroscopy (UV/VIS), chromatography (HPLC), NMR and/or mass spectrometry.

All carotenoids can be natural or nature identical. Nature identical carotene is a synthetic carotene which has exactly the same chemical structure as natural carotene found in nature.

The emulsion of the present invention has oil droplet size (using dynamic light scattering) of 50 - 400 nm, preferably 80 - 300 nm, more preferably 100 - 150 nm, especially 130 - 140 nm.

According to the invention the oil droplet size is the z-average size, measured in one embodiment using a Malvern zetasizer nano-s (serial nr. MAL500613).

The emulsion of the present invention has in one alternative polydispersity index (PDI) between 0.01 and 0.4, preferably between 0.01 and 0.3, more preferably between 0.01 and 0.2, especially between 0.01 and 0.1. Polydispersity index (PDI) defines the width of the oil droplet size distribution and is defined in ISP13321 part 8.
The emulsion of the present invention has in one alternative 70% of its oil droplets between 0 and 200 nm, preferably 80% of its oil droplets between 0 and 200 nm, more preferably 90% of its oil droplets between 0 and 200 nm, especially 95% of its oil droplets between 0 and 200 nm.

In one embodiment the present emulsion has a colour intensity value (E 1/1) of 140 - 280, preferably 140 - 250, 150 - 250, more preferably 150 - 230, 150 - 220, more preferably 160 - 230, 180 - 215, especially 205 - 215,

The colour intensity value of aqueous solutions (E 1/1) is defined as the absorbance of light at maximum absorbance (different for each carotenoid) going through 1 cm cuvette containing 1% carotenoid emulsion. If the colour intensity value (E 1/1) is measured at lower concentration than 1%, the measured value of the colour intensity has to be corrected with a dilution factor. $E 1 / 1 = \left({A}_{max}\times 20\right) / \left(weight of sample \left(g\right)\right)$

In one embodiment of the present invention the carotenoid is melt and/or solved and/or isomerized from trans to cis in triacylglycerol oil, such as MCT oil (medium-chain triacylglycerol), olive oil, corn oil, sunflower oil, peanut oil, soy oil or other alternative vegetable oil, preferably MCT oil.

Accordingly, the emulsions of the invention comprise in one embodiment triacylglycerol oil, selected from the group consisting of: MCT oil (medium-chain triacylglycerol), olive oil, sunflower oil, peanut oil, soy oil and vegetable oil, preferably MCT oil in a concentration of 0.1-150 ppm, preferably 1-75 ppm, more preferably 5-50 ppm, especially 15-30 ppm.

The triacylglycerol oil used in the present invention is in one embodiment an ester of glycerol where glycerol is esterified to a fatty acid where the fatty acid can have 4-22 carbon chain length and double bond on any of the carbon positions.
Preferably MCT oil is used, where the MCT is an ester of glycerol where glycerol is esterified to a fatty acid where the fatty acid are saturated and have 6-10 carbon chain length, preferably 8 - 10 carbon chain length.
The isomerization is carried out for an adequate time at a temperature of 100 - 200°C, 120 - 180°C, more preferably 130 - 160°C, till the trans-isomer content is between 40 and 75%.

The isomerization can be carried out in the presence of an oil soluble antioxidant.

In one embodiment of the invention the emulsions comprise an oil-soluble antioxidant in a concentration of 0.001-60 ppm, preferably 0.01-30 ppm, more preferably 0.1-20 ppm especially 0.5-12 ppm.
According to one embodiment of the present invention oil-soluble antioxidants are soluble in non-polar solvents and form a molecular dispersion.
Oil-soluble antioxidants according to the invention are selected from the group consisting of:
- Ethoxyquin,
- hindered phenolic antioxidants, such as t-butylhydroxytoluol, t- butylhydroxyaniso, t-butylhydroxyquinone;
- Vitamin A, retinoic acid and its esters with C1-C20 carbon chain length,
- Vitamin D2 and D3,
- alpha, beta, gamma, and delta Tocopherol or mixtures comprising at least two of the tocoperols;
- alpha, beta, gamma, and delta Tocotrienols or mixtures comprising at least two of the tocotrienols;
- natural extracts comprising at least one of the above compounds, phenolic diterpenes such as Carnosol, Carnosic acid;
- derivatives of cinnamic acid like 2-ethoxyethyl p-methoxycinnamate, ethylhexyl p-methoxycinnamate, 2-ethylhexyl 4-methoxycinnamate, methyl diisopropylcinnamate, isoamyl 4-methoxycinnamate, diethanolamin 4-methoxycinnamate and such as BASF products Tinogard TT, Tinogard HS, LC-gallates, Eugenol, Thymol, Organosolv-Lignin,
- preferably alpha-tocopherol,
- whereby the oil soluble antioxidant is in a concentration of 0.001-60 ppm, preferably 0.1-30 ppm, more preferably 1-20 ppm especially 6-12 ppm.

By using the process of the present invention the carotenoid shows a high stability solved in the triacylglycerol oil, so that no solid particles are within the droplets. The droplets are a mixture of carotenoid, optionally oil-soluble antioxidant and triacylglycerol oil.
In one embodiment the mixture of carotenoid, optionally oil-soluble antioxidant and triacylglycerol oil is a solution (meaning a molecular dispersion of the carotenoid, optionally oil-soluble antioxidant and triacylglycerol oil).
In one embodiment of the invention the carotene can be crystallized or partly crystallized.

In the invention the emulsion comprises an octenyl succinate starch.
The modified starch has a concentration of 0.5-600 ppm, preferably 2-300 ppm, more preferably 20-200 ppm, especially 60-120 ppm. The above mentioned polymers have a molecular weight MW -distribution of 10.000 - 2.000.000 g/mol, preferably 20.000 - 1.000.000 g/mol, more preferably 30.000 - 500.000 g/mol.
In an embodiment the emulsion of the invention is free from octenyl-succinic anhydride-modified gum acacia (gum acacia is also known as gum arabic).

The emulsion comprises a carbohydrate selected from the group consisting of glucose-syrup, maltose and trehalose, preferably glycose syrup, and said carbohydrates have a DE (dextrose equivalent) between 20 and 50, preferably between 35 and 50 especially between 43 and 48.
Said carbohydrate has a concentration of 0.5-10000 ppm, preferably 2-5000 ppm, more preferably 20-3000 ppm, especially 60-2000 ppm.

The emulsions of the present invention comprise in one alternative at least one water-soluble antioxidant selected from the group consisting of:
- natural compounds that are active as antioxidants because they comprise a phenolic OH-group in their chemical structure: like hydroxy derivatives of cinnamic acid, e.g. hydroxycinnamic acids, hydroxycinnamates, which are a class of polyphenols having a C6-C3 skeleton, for example hydroxyhydrocinnamate;
- caffeic acid, ferulic acid, tyrosol, hydroxytyrosol, cinnamic acid, chlorogenic acid, coumarin, coumarinic acid, sinapic acid, cinnamic acid, chicoric acid, and esters of any of these compounds with C1-C20;
- extracts of plants rich in at least one of the above compounds;
- rosmarinic acid, hydroxytyrosol;
- extracts from common spices. In one embodiment common spices are selected from the group comprising rosemary, lemon balm, oregano, thyme, peppermint, sage or similar plants comprising or being rich in at least one of the above compounds;
- flavons, which are a class of natural compounds of which more than 5000 exist, used as antioxidants can be any of them as extracted from plants such as tea or any other plant that comprise or is rich in catechin or epicatechin or derivatives, whereby these compounds can be glycosylated with carbohydrates or esterified with fatty acids C1-C20 or gallic acid; extracts from plants such as tea, olives, pears, apples comprising or being rich in one or more of the above mentioned compounds;
- sodium ascorbate, polyphenole, Teanova 80, glutathione, lipoic acid, catechin, punicalagin, xanthone, benzotropolones, preferably sodium ascorbate;
- whereby the water soluble antioxidant is in a concentration of 0.001-60 ppm, preferably 0.1-30 ppm, more preferably 1-20 ppm, especially 6-12 ppm.

According to one embodiment of the present invention water-soluble antioxidants form a molecular dispersion in water.

Subject matter of the present invention is also a process for manufacturing the emulsion comprising the following steps:
a) manufacturing a solution comprising octenyl succinate starch in a concentration of 0,7 - 70 %, and at least one carbohydrate selected from the group consisting of: glucose-syrup, maltose and trehalose, said carbohydrates having a DE between 20 and 50 in a concentration of 0,001 - 80 % and water and mixing the components,
b) manufacturing a solution by mixing at least one carotenoid in a concentration of 0,1 - 15 %, preferably 0.5 - 5 %, more preferably 0.5 - 3 %, especially 1 - 2 %, in triacylglycerol oil in a concentration of 1 - 30 %, preferably 3 - 20 %, more preferably 3 - 15 %, 3 - 10 %, especially 3 - 5 %, and melting the dispersed carotenoid at a temperature of 100- 200°C and isomerizing the at least one carotenoid in triacylglycerol oil until a trans-isomer content is between 40% and 75% and,
c) introducing the solution of step b) into solution of step a),
d) performing at least one step of pre-emulsification by passing the mixture of step c) through a rotator/stator mixer,
e) performing at least one step, preferably two steps, of high pressure homogenization, preferably at a pressure of 300 - 2.000 bar,
f) spray drying the emulsion of step e) to obtain a powder,
g-1) manufacturing an emulsion by introducing the powder of step f) in water;
whereby the percent are weight percent referring to the emulsion of step c) to e) and the total amount of all used compounds is 100%.

A further subject matter is a process for manufacturing an oil-in-water emulsion comprising the steps a) to f) as mentioned above and manufacturing in step g-2) replacing step g-1) an emulsion by introducing the powder of step f) in water in a concentration of 5% - 70%, preferably 10% - 70%, 10% - 60%, more preferably 20% - 50%, whereby the percent are weight percent referring to the concentration of the powder according to step f) in water.

In one embodiment of the process of the invention optionally at least one water soluble antioxidant in a concentration of 0,001 - 10 %, preferably 0,001 - 4 %, more preferably 0,01 - 1.5 %, especially 0.5 - 1.5 is added in step a).

In one embodiment of the process of the invention optionally at least one oil-soluble antioxidant in a concentration of 0,001 - 10 %, preferably 0,001 - 5 %, more preferably 0,01 - 2 %, especially 0.1 - 1 %, in triacylglycerol oil in a concentration of 1 - 30 %, preferably 3 - 20 %, more preferably 3 - 15 %, 3 - 10 %, especially 3 - 5 %, is added in step b).

In one alternative the process of the invention comprises one or more of the alternatives comprising:
- adding the water soluble antioxidant after step c), d) or e),
- adding the oil soluble antioxidant in step a), c) or d).

In one embodiment the pre-emulsification is carried out till the oil droplet size (z-average size) is 200 nm to 20 µm, preferably 250 nm to 10 µm, more preferably 300 nm to 5 µm.

The pre-emulsion is stable for at least 10 hours at 60°C (with stirring), preferably 5 hours at 60°C (with stirring), more preferably 3 hours at 60°C (with stirring).

In one embodiment a two-stage homogenizer is used in the step of high pressure homogenization. The two-stage homogenizer consists of two pressure valves and therefore each pass though the machine consists of two stages. The first stage with 0-3000 bar, preferably 500-2000 bar, more preferably 650-1500 bar, especially 800-950 bar; and the second stage with 0-3000 bar, preferably 50-2000 bar, more preferably 50-1500 bar, especially 50-1000 bar.
In an alternative the emulsion is passed in one stage through a microfluidizer with 300-2000 bar, preferably 500-1500 bar, more preferably 500-1400 bar.

All steps in the process are conducted at temperatures between 0 and 500°C, preferably 20 - 200°C, more preferably 20-200°C especially 50-200°C.
The step of high pressure homogenization is carried out in an alternative for at least 2, 3, 4, 5, 6, 7, 8, 9, 10, 15, 20, 30 or even more.

The stable oil-in-water emulsion of the invention is free from other surfactants and/or emulsifiers than octenyl succinate starch. In other words, octenyl succinate starch is the only surfactant and/or emusifier present in the emulsion of the invention.
Further subject matter of the present invention is the powder comprising a carotenoid according to step f).
The powder has in one alternative a residual moisture content of 0-15%, preferably 0.1-10%, more preferably 3-5%.
In one embodiment the powder produced according to the process of the invention comprises:
- an octenyl succinate starch in a concentration of 0,7 - 70 %, preferably 1 - 60 %, more preferably 10 - 50 %, especially 20 - 40 %,
- optionally at least one water soluble antioxidant in a concentration of 0,001 - 10 %, preferably 0,001 - 4 %, more preferably 0,001 - 2 %, especially 1 %,
- at least one carbohydrate selected from the group consisting of: glucose-syrup, maltose and trehalose, said carbohydrates having a DE between 20 and 50 in a concentration of 0,001 - 80 %, preferably 10 - 80 %, more preferably 20 - 70 %, especially 45 %,
- at least one carotenoid in a concentration of 0,1 - 15 % with a trans-isomer content between 40 and 75%,
- optionally at least one oil-soluble antioxidant in a concentration of 0,001 - 10 %, preferably 0,001 - 5 %, more preferably 0,01 - 3 %, especially 1 %,
- a triacylglycerol oil in a concentration of 1 - 30 %,
- and the total amount of all used compounds is 100%.

In one embodiment of the invention the powder is obtained by spray drying at inlet temperatures between 80°C and 500°C, preferably 100-300°C, more preferably 100-200°C and an outlet temperature of about 40-150°C, preferably 50-100°C, more preferably 50-90°C.

Another subject matter of the present disclosure is the (concentrated) emulsion comprising a carotenoid according to step g-2), manufactured by introducing the powder of step f) in water. This (concentrated) emulsion is a stock-emulsion which can be further diluted.

In one embodiment of the disclosure the aqueous, transparent emulsion, the concentrated stock-emulsion and/or the powder of the invention are used as colorant, preferably natural or naturally identical colorant. The aqueous emulsion, the concentrated stock-emulsion and/or the powder of the invention are used as colorant in beverages like soft drinks, flavoured water, fruit juices, punches or concentrated forms of these beverages but also alcoholic beverages and instant beverage powders.

In a further embodiment the aqueous emulsion, the concentrated stock-emulsion and/or the powder of the invention are used in food and/or feed. Typically, the aqueous emulsion, the concentrated stock-emulsion and/or the powder of the invention are used in ice cream, cheese, milk product like milk drinks or yoghurt, soy milk and the like, confectionary products, gums, dessert, candies, puddings, jellies, instant pudding powder, but also in snacks, cookies, sauces, cereals, salad dressing, soups.

The aqueous solution, the concentrated stock-emulsion and/or the powder of the invention can also be used in pharmaceutical preparations, such as tablets or capsules, or cosmetic and dermal products.

In one embodiment the aqueous emulsion, the concentrated stock-emulsion and/or the powder of the invention are used instead of azo dyes and replace them. The products of the invention replace azo-dyes as food colorant, by replicating the visual appearance of the end product, meaning the final products have the same colour and transparency.

### Examples:

### 1. Preparation procedure

a) The modified starch, Na-ascorbic acid and the carbohydrate were dissolved in water having a temperature of 60°C.
b) Apocarotenal, including tocopherol was solved and isomerized in MCT-oil at 130-160°C for 4 minutes.
c) The solution of step b) was mixed with the solution of step a)
d) The mixture of step c) was pre-emulsified in a lab scale rotator/stator mixer for 9 min.
e) The mixture was further emulsified by a high pressure microfluidyzer.
f) The emulsion was spray dried. The inlet temperature was 100 to 120°C. The outlet temperature was about 60°C.
g) An emulsion was manufactured by introducing the dry powder in water at a concentration of carotenoid of 5 ppm.

The powder had residual moisture content in a concentration of 5%.

In the following example the E1/1 value was measured at 460 nm maximum extinction, maximum extinction of 1% apocarotenal in solvent was 2640. Furthermore, absorbance at 600 nm is an indication of turbidity.

### 2. Increasing Apocarotenal concentration

An emulsion according to example 1 was manufactured with the following compounds and concentration, whereby the mixture of step 1 e) was further emulsified by a high pressure microfluidyzer (1 pass at 1000 bar):

| | 2% apocarotenal | 3% apocarotenal | 4% apocarotenal | 5% apocarotenal |
|---|---|---|---|---|
| Apocarotenal (%) | 2 | 3 | 4 | 5 |
| MCT oil (%) | 8 | 12 | 16 | 20 |
| Glucose syrup (%) | 48 | 38 | 34 | 30 |
| Modified starch (%) | 40 | 40 | 40 | 40 |
| Na Ascorbate (%) | 1 | 1 | 1 | 1 |
| Tocopherol (%) | 1 | 1 | 1 | 1 |
| Particle size (nm) | 126 | 142 | 148 | 155 |
| Absorbance at 600 nm | 0,016 | 0,03 | 0,05 | 0,06 |
| E1/1 value | 208 | 206 | 204 | 201 |
| Turbidity NTU | 17 | 31 | 33,7 | 34 |

### 3. Using various carbohydrates

An emulsion according to example 1 was manufactured with the following compounds and concentration, whereby the mixture of step 1 e) was further emulsified by a high pressure microfluidyzer (1 pass at 1000 bar):

| Trehalose as carbohydrate | | Glucose syrup as carbohydrate | | Glucidex 47 as carbohydrate | |
|---|---|---|---|---|---|
| Apocarotenal (%) | 2 | Apocarotenal (%) | 2 | Apocarotenal (%) | 2 |
| MCT oil (%) | 8 | MCT oil (%) | 8 | MCT oil (%) | 8 |
| Trehalose (%) | 48 | Glucose syrup (%) | 48 | Glucidex 47 (%) | 48 |
| Modified starch (%) | 38 | Modified starch (%) | 38 | Modified starch (%) | 38 |
| Na Ascorbate (%) | 1 | Na Ascorbate (%) | 1 | Na Ascorbate (%) | 1 |
| Tocopherol (%) | 1 | Tocopherol (%) | 1 | Tocopherol (%) | 1 |
| Particle size (nm) | 140 | Particle size (nm) | 130 | Particle size (nm) | 111 |
| Absorbance at 600 nm | 0,022 | Absorbance at 600 nm | 0,02 | Absorbance at 600 nm | 0,016 |
| E1/1 value | 208 | E1/1 value | 206 | E1/1 value | 207 |

### 4. Using various modified starch concentration

An emulsion according to example 1 was manufactured with the following compounds and concentration, whereby the mixture of step 1 e) was further emulsified by a high pressure microfluidyzer (1 pass at 800 bar):

| | 10% starch | 20% starch | 40% starch |
|---|---|---|---|
| Apocarotenal (%) | 2 | 2 | 2 |
| MCT oil (%) | 8 | 8 | 8 |
| Glucose syrup (%) | 75 | 65 | 45 |
| Modified starch (%) | 10 | 20 | 40 |
| Na Ascorbate (%) | 1 | 0 | 0 |
| Tocopherol (%) | 1 | 1 | 1 |
| Particle size (nm) | 113 | 134 | 131 |
| Absorbance at 600 nm | 0,018 | 0,022 | 0,022 |
| E1/1 value | 206 | 206 | 206 |
| Turbidity NTU | 23,9 | 19 | 18,7 |

### 5. Using various pressures

An emulsion according to example 1 was manufactured with the following compounds and concentration, whereby the mixture of step 1 e) was further emulsified by a high pressure microfluidyzer (1 pass at 600 or 1000 bar):

| | 600 bar | 800 bar | 1000 bar |
|---|---|---|---|
| Apocarotenal (%) | 2 | 2 | 2 |
| MCT oil (%) | 8 | 8 | 8 |
| Glucose syrup (%) | 48 | 48 | 48 |
| Modified starch (%) | 38 | 38 | 38 |
| Na Ascorbate (%) | 0 | 0 | 0 |
| Tocopherol (%) | 1 | 1 | 1 |
| Particle size (nm) | 150 | 131 | 123 |
| Absorbance at 600 nm | 0,036 | 0,022 | 0,019 |
| E1/1 value | 207 | 206 | 207 |

### 6. Passing 1-5 times through the microfluidyzer

An emulsion according to example 1 was manufactured with the following compounds and concentration, whereby the mixture of step 1 e) was further emulsified by a high pressure microfluidyzer (at 1000 bar):

| | |
|---|---|
| Apocarotenal (%) | 1 |
| MCT oil (%) | 4 |
| Glucose syrup (%) | 49 |
| Modified starch (%) | 40 |
| Na Ascorbate (%) | 2 |
| Tocopherol (%) | 1 |
| Particle size 1 pass (nm) | 150 |
| Particle size 2 pass (nm) | 114 |
| Particle size 3 pass (nm) | 101 |
| Particle size 4 pass (nm) | 104 |
| Particle size 5 pass (nm) | 102 |

## Claims

1. Aqueous transparent oil-in-water emulsion consisting of
- oil-droplets in water, of an emulsified carotenoid selected from the group consisting of cantaxanthin, astaxanthin, lutein, zeaxanthin, beta-zeacaroten, lycopen, apocarotenal, bixin, paprika olioresin, capsanthin and capsorubin in a concentration of 0.025-2000 ppm,
wherein 40 to 75% of the carotenoid is the trans-isomer;
whereby the carotenoid is present in the droplets with an oil droplets size measured with dynamic light scattering of 50 - 400 nm and solved in the oil, the oil being a triacylglycerol oil,
- octenyl succinate starch in a concentration of 0.5-600 ppm, and
- at least one carbohydrate selected from the group consisting of:
glucose-syrup, maltose and trehalose, said carbohydrates having a DE between 20 and 50 in a concentration of 0.5-10000 ppm,
- optionally
at least one water-soluble antioxidant selected from the group consisting of:
- natural compounds that are active as antioxidants by comprising a phenolic OH-group in their chemical structure;
- caffeic acid, ferulic acid, tyrosol, hydroxytyrosol, cinnamic acid, chlorogenic acid, coumarin, coumarinic acid, sinapic acid, cinnamic acid, chicoric acid, and esters of any of these compounds with C1-C20;
- extracts of plants rich in at least one of the above compounds;
- rosmarinic acid, hydroxytyrosol;
- extracts from common spices;
- flavons, preferably extracted from plants such as tea or any other plant that comprising catechin or epicatechin or derivatives;
- extracts from plants such as tea, olives, pears, apples comprising one or more of the above mentioned compounds;
- sodium ascorbate, polyphenole, Teanova 80, glutathione, lipoic acid, catechin, punicalagin, xanthone, benzotropolones, preferably sodium ascorbate, whereby the water soluble antioxidant is in a concentration of 0.001-60 ppm
and/or
at least one oil-soluble antioxidant selected from the group consisting of:
- ethoxyquin,
- hindered phenolic antioxidants, such as t-butylhydroxytoluol, t-butylhydroxyanisol, t-butylhydroxyquinone;
- Vitamin A, retinoic acid and its esters with C1-C20;
- Vitamin D2 and D3;
- alpha, beta, gamma, and delta Tocopherol or mixtures comprising at least two of the tocopherols;
- alpha, beta, gamma, and delta Tocotrienols or mixtures comprising at least two of the tocotrienols;
- natural extracts comprising at least one of the above compounds,
- phenolic diterpenes such as Carnosol, Carnosic acid;
- derivatives of cinnamic acid like 2-ethoxyethyl p-methoxycinnamate, ethylhexyl p-methoxycinnamate, 2-ethylhexyl 4-methoxycinnamate, methyl diisopropylcinnamate, isoamyl 4-methoxycinnamate, diethanolamin 4-methoxycinnamate and
- LC-gallates, Eugenol, Thymol,
whereby the oil soluble antioxidant is in a concentration of 0.001-60 ppm,
wherein the emulsion has a NTU (Turbidity) value at a concentration of 5 ppm of carotenoid of 1 - 35 NTU,
wherein NTU is nephelometric turbidity units that are measured with any standard turbidimeter,
wherein the oil droplet size is the z-average size.

2. Emulsion according to claim 1 whereby the carotenoid is apocarotenal, more preferably beta-apo-8-carotenal.

3. Emulsion according to any of the preceding claims with a colour intensity value (E 1/1) of 140 - 280, whereby the colour intensity value (E 1/1) is defined as the absorbance of light at maximum absorbance going through 1 cm cuvette containing 1% carotene dissolved in organic solvent.

4. Emulsion according to any of the preceding claims comprising a triacylglycerol oil selected from the group consisting of: MCT oil (medium-chain triacylglycerol), olive oil, sunflower oil, peanut oil, soy oil and vegetable oil, preferably MCT oil, in a concentration of 0.1-150 ppm.

5. Process for manufacturing the emulsion according to any of the claims 1 to 4 comprising the following steps:
a) manufacturing a solution comprising octenyl succinate starch in a concentration of 0,7 - 70 %, and at least one carbohydrate selected from the group consisting of: glucose-syrup, maltose and trehalose, said carbohydrates having a DE between 20 and 50 in a concentration of 0,001 - 80 % and water and mixing the components,
b) manufacturing a solution by mixing at least one carotenoid in a concentration of 0,1 - 15 % in triacylglycerol oil in a concentration of 1 - 30 % and melting the dispersed carotenoid at a temperature of 100-200°C and isomerizing the at least one carotenoid in triacylglycerol oil until a trans-isomer content is between 40% and 75% and,
c) introducing the solution of step b) into solution of step a),
d) performing at least one step of pre-emulsification by passing the mixture of step c) through a rotator/stator mixer,
e) performing at least one step of high pressure homogenization,
f) spray drying the emulsion of step e) to obtain a powder,
g-1) manufacturing an emulsion by introducing the powder of step f) in water; whereby the percent are weight percent referring to the emulsion of step c) to e),
or
g-2) manufacturing an emulsion by introducing the powder of step f) in water in a concentration of 5% - 70% whereby the percent are weight percent referring to the concentration of the powder according to step f) in water.

6. The process of claim 5 comprising one or more of the alternatives comprising:
- adding the water soluble antioxidant after step c), d) or e),
- adding the oil soluble antioxidant in step a), c) or d).

7. The powder comprising a carotenoid according to step f) of claim 5.

8. Use of the powder of claim 7 as colorant, preferably natural or nature identical colorant, more preferably as replacement for azo dyes, in food, feed, pharmaceutical preparations, cosmetic and/or dermal products.

## Patentansprüche

1. Wässrige transparente Öl-in-Wasser-Emulsion, bestehend aus:
- Öltröpfchen in Wasser aus einem emulgierten Carotenoid ausgewählt aus der Gruppe bestehend aus Cantaxanthin, Astaxanthin, Lutein, Zeaxanthin, beta-Zeacaroten, Lycopen, Apocarotenal, Bixin, Paprika-Olioresin, Capsanthin und Capsorubin in einer Konzentration von 0,025-2000 ppm,
wobei 40 bis 75 % des Carotenoids das trans-Isomer sind; wobei das Carotenoid in den Tröpfchen, die eine durch dynamische Lichtstreuung gemessene Öltröpfchengröße von 50-400 nm aufweist, und in dem Öl gelöst vorliegt, wobei das Öl ein Triacylglycerolöl ist,
- Octenylsuccinatstärke in einer Konzentration von 0,5-600 ppm und
- wenigstens einem Kohlenhydrat ausgewählt aus der Gruppe bestehend aus: Glucosesirup, Maltose und Trehalose, wobei die Kohlenhydrate einen DE-Wert von zwischen 20 und 50 aufweisen, in einer Konzentration von 0,5-10000 ppm,
- gegebenenfalls
wenigstens einem wasserlöslichen Antioxidationsmittel ausgewählt aus der Gruppe bestehend aus:
- natürlichen Verbindungen, die als Antioxidationsmittel wirksam sind, indem sie eine phenolische OH-Gruppe in ihrer chemischen Struktur umfassen;
- Caffeinsäure, Ferulsäure, Tyrosol, Hydroxytyrosol, Zimtsäure, Chlorogensäure, Cumarin, Cumarinsäure, Sinapinsäure, Zimtsäure, Zichoriensäure und Estern beliebiger dieser Verbindungen mit C1-C20;
- Extrakten von Pflanzen, die reich an wenigstens einer der vorstehenden Verbindungen ist;
- Rosmarinsäure, Hydroxytyrosol;
- Extrakten von verbreiteten Gewürzen;
- Flavonen, vorzugsweise extrahiert aus Pflanzen, wie z. B. Tee oder jeder anderen Pflanze, die Catechin oder Epicatechin oder Derivate umfasst;
- Extrakten von Pflanzen, wie z. B. Tee, Oliven, Birnen, Äpfel, die eine oder mehrere der vorstehend genannten Verbindungen umfassen;
- Natriumascorbat, Polyphenol, Teanova 80, Glutathion, Liponsäure, Catechin, Punicalagin, Xanthon, Benzotropolone, vorzugsweise Natriumascorbat,
wobei das wasserlösliche Antioxidationsmittel in einer Konzentration von 0,001-60 ppm vorliegt,
und/oder
wenigstens einem öllöslichen Antioxidationsmittel ausgewählt aus der Gruppe bestehend aus:
- Ethoxyquin,
- Gehindertes-Phenol-Antioxidationsmitteln, wie z. B. t-Butylhydroxytoluol, t-Butylhydroxyanisol, t-Butylhydroxychinon;
- Vitamin A, Retinolsäure und ihren Estern mit C1-C20;
- Vitamin D2 und D3;
- alpha-, beta-, gamma- und delta-Tocopherol oder Gemischen, die wenigstens zwei der Tocopherole umfassen;
- alpha-, beta-, gamma- und delta-Tocotrienolen oder Gemischen, die wenigstens zwei der Tocotrienole umfassen;
- natürlichen Extrakten, die wenigstens zwei der vorstehenden Verbindungen umfassen;
- phenolischen Diterpenen, wie z. B. Carnosol, Carnosinsäure;
- Derivaten von Zimtsäure, wie z. B. 2-Ethoxyethyl-p-methoxycinnamat, Ethylhexyl-p-methoxycinnamat, 2-Ethylhexyl-4-methoxycinnamat, Methyldiisopropylcinnamat, Isoamyl-4-methoxycinnamat, Diethanolamin-4-methoxycinnamat; und
- LC-Gallaten, Eugenol, Thymol,
wobei das öllösliche Antioxidationsmittel in einer Konzentration von 0,001-60 ppm vorliegt,
wobei die Emulsion einen NTU(Trübungs)-Wert bei einer Konzentration von 5 ppm Carotenoid von 1-35 NTU aufweist, wobei NTU nephelometrische Trübungseinheiten sind, die mit einem beliebigen Standard-Turbidimeter gemessen sind, wobei die Öltröpfchengröße die z-gemittelte Größe ist.

2. Emulsion gemäß Anspruch 1, wobei das Carotenoid Apocarotenal ist, bevorzugter beta-Apo-8-carotenal.

3. Emulsion gemäß einem der vorstehenden Ansprüche mit einem Farbintensitätswert (E 1/1) von 140-280, wobei der Farbintensitätswert (E 1/1) als die Absorbanz von Licht bei der maximalen Absorbanz, das durch eine 1-cm-Küvette tritt, die 1 % Caroten in organischem Lösungsmittel gelöst enthält, definiert ist.

4. Emulsion gemäß einem der vorstehenden Ansprüche, umfassend ein Triacylglycerolöl ausgewählt aus der Gruppe bestehend aus: MCT-Öl (Triacylglycerol mit mittlerer Kettenlänge), Olivenöl, Sonnenblumenöl, Erdnussöl, Sojaöl und Pflanzenöl, vorzugsweise MCT-Öl, in einer Konzentration von 0,1-150 ppm.

5. Verfahren zur Herstellung der Emulsion gemäß einem der Ansprüche 1 bis 4, umfassend die folgenden Schritte:
a) Herstellen einer Lösung, die Octenylsuccinatstärke in einer Konzentration von 0,7-70 % und wenigstens ein Kohlenhydrat ausgewählt aus der Gruppe bestehend aus: Glucosesirup, Maltose und Trehalose, wobei die Kohlenhydrate einen DE-Wert von zwischen 20 und 50 aufweisen, in einer Konzentration von 0,001-80 % und Wasser umfasst, und Mischen der Komponenten,
b) Herstellen einer Lösung durch Mischen wenigstens eines Carotenoids in einer Konzentration von 0,1-15 % in Triacylglycerolöl in einer Konzentration von 1-30 % und Schmelzen des dispergierten Carotenoids bei einer Temperatur von 100-200 °C und Isomerisieren des wenigstens einen Carotenoids in Triacylglycerolöl, bis ein Trans-Isomergehalt zwischen 40 % und 75 % beträgt, und
c) Einführen der Lösung von Schritt b) in die Lösung von Schritt a),
d) Durchführen wenigstens eines Schritts von Voremulgierung durch Führen des Gemischs von Schritt c) durch einen Rotor/Stator-Mischer,
e) Durchführen wenigstens eines Schritts von Hochdruckhomogenisierung,
f) Sprühtrocknen der Emulsion von Schritt e), um ein Pulver zu erhalten,
g-1) Herstellen einer Emulsion durch Einführen des Pulvers von Schritt f) in Wasser; wobei die Prozentwerte Gewichtsprozent bezogen auf die Emulsion von Schritt c) bis e) sind,
oder
g-2) Herstellen einer Emulsion durch Einführen des Pulvers von Schritt f) in Wasser in einer Konzentration von 5 % bis 70 %, wobei die Prozentwerte Gewichtsprozent bezogen auf die Konzentration des Pulvers gemäß Schritt f) in Wasser sind.

6. Verfahren gemäß Anspruch 5, umfassend eine oder mehrere der Alternativen umfassend:
- Zugeben des wasserlöslichen Antioxidationsmittels nach Schritt c), d) oder e),
- Zugeben des öllöslichen Antioxidationsmittels bei Schritt a), c) oder d).

7. Pulver, umfassend ein Carotenoid gemäß Schritt f) von Anspruch 5.

8. Verwendung des Pulvers gemäß Anspruch 7 als Farbmittel, vorzugsweise natürliches oder naturidentisches Farbmittel, bevorzugter als Ersatz für Azofarbstoffe, in Lebensmitteln, Futtermitteln, pharmazeutischen Zubereitungen, kosmetischen und/oder Hautprodukten.

## Revendications

1. Émulsion transparente aqueuse huile-dans-eau constituée de :
- gouttelettes d'huile dans de l'eau, constituées d'un caroténoïde émulsifié sélectionné dans le groupe constitué de la cantaxanthine, de l'astaxanthine, de la lutéine, de la zéaxanthine, du bêta-zéacarotène, du lycopène, de l'apocaroténal, de la bixine, d'une oléorésine de paprika, de la capsanthine et de la capsorubine, dans une concentration de 0,025 à 2 000 ppm, 40 à 75 % du caroténoïde étant le trans-isomère ;
le caroténoïde étant présent dans les gouttelettes, les gouttelettes ayant une taille de gouttelette d'huile, mesurée par diffusion dynamique de la lumière, de 50 à 400 nm, et étant dissous dans l'huile, l'huile étant une huile de triacylglycérol,
- octénylsuccinate d'amidon dans une concentration de 0,5 à 600 ppm, et
- au moins un glucide sélectionné dans le groupe constitué de : sirop de glucose, maltose et tréhalose, lesdits glucides ayant une valeur d'équivalent dextrose (DE) de 20 à 50 dans une concentration de 0,5 à 10 000 ppm,
- optionnellement
au moins un antioxydant hydrosoluble sélectionné dans le groupe constitué de :
- composés naturels qui sont actifs comme antioxydants du fait qu'ils comprennent un groupe OH phénolique dans leur structure chimique ;
- acide caféique, acide férulique, tyrosol, hydroxytyrosol, acide cinnamique, acide chlorogénique, coumarine, acide coumarinique, acide sinapique, acide cinnamique, acide chicorique, et esters C₁-C₂₀ de n'importe lesquels de ces composés ;
- extraits de plantes riches en au moins l'un des composés ci-dessus ;
- acide rosmarinique, hydroxytyrosol ;
- extraits d'épices communes ;
- flavones, préférablement extraites de plantes telles que le thé ou toute autre plante comprenant de la catéchine ou de l'épicatéchine ou leurs dérivés ;
- extraits de plantes telles que le thé, les olives, les poires, les pommes, comprenant un ou plusieurs des composés susmentionnés ;
- ascorbate de sodium, polyphénols, Teanova 80, glutathion, acide lipoïque, catéchine, punicalagine, xanthone, benzotropolones, préférablement l'ascorbate de sodium,
l'antioxydant hydrosoluble étant présent dans une concentration de 0,001 à 60 ppm ;
et/ou
au moins un antioxydant liposoluble sélectionné dans le groupe constitué de :
- éthoxyquine,
- antioxydants phénoliques encombrés, tels que le t-butylhydroxytoluol, le t-butylhydroxyanisol, la t-butylhydroxyquinone ;
- vitamine A, acide rétinoïque et ses esters C₁-C₂₀ ;
- vitamines D2 et D3 ;
- alpha-, bêta-, gamma- et delta-tocophérol, ou mélanges comprenant au moins deux des tocophérols ;
- alpha-, bêta-, gamma- et delta-tocotriénol ou mélanges comprenant au moins deux des tocotriénols ;
- extraits naturels comprenant au moins l'un des composés ci-dessus, diterpènes phénoliques tels que le carnosol, l'acide carnosique ;
- dérivés de l'acide cinnamique comme le p-méthoxycinnamate de 2-éthoxyéthyle, le p-méthoxycinnamate d'éthylhexyle, le 4-méthoxycinnamate de 2-éthylhexyle, le diisopropylcinnamate de méthyle, le 4-méthoxycinnamate d'isoamyle, le 4-méthoxycinnamate de diéthanolamine ; et
- LC-gallates, eugénol, thymol,
l'antioxydant liposoluble étant présent dans une concentration de 0,001 à 60 ppm,
l'émulsion ayant une valeur NTU (turbidité), à une concentration de 5 ppm de caroténoïde, de 1 à 35 NTU,
le terme NTU signifiant des unités de turbidité néphélométrique qui sont mesurées avec n'importe quel turbidimètre standard,
la taille de gouttelette d'huile étant la taille moyenne en z.

2. Émulsion selon la revendication 1, dans laquelle le caroténoïde est l'apocaroténal, plus préférablement le bêta-apo-8-caroténal.

3. Émulsion selon l'une quelconque des revendications précédentes, ayant une valeur d'intensité de couleur (E 1/1) de 140 à 280, la valeur d'intensité de couleur (E 1/1) étant définie comme l'absorbance de la lumière à un niveau maximal d'absorbance traversant une cuvette de 1 cm contenant 1 % de carotène dissous dans un solvant organique.

4. Émulsion selon l'une quelconque des revendications précédentes, comprenant une huile de triacylglycérol sélectionnée dans le groupe constitué de : huile de TCM (triacylglycérol à chaîne moyenne), huile d'olive, huile de tournesol, huile d'arachide, huile de soja et huile végétale, préférablement huile MCT, dans une concentration de 0,1 à 150 ppm.

5. Procédé de fabrication de l'émulsion selon l'une quelconque des revendications 1 à 4, comprenant les étapes suivantes :
a) la fabrication d'une solution comprenant de l'octénylsuccinate d'amidon dans une concentration de 0,7 à 70 %, et au moins un glucide sélectionné dans le groupe constitué de : sirop de glucose, maltose et tréhalose, lesdits glucides ayant une valeur d'équivalent dextrose (DE) de 20 à 50 dans une concentration de 0,001 à 80 % et de l'eau, et le mélange des constituants,
b) la fabrication d'une solution en mélangeant au moins un caroténoïde dans une concentration de 0,1 à 15 % dans de l'huile de triacylglycérol dans une concentration de 1 à 30 % et en faisant fondre le caroténoïde dispersé à une température de 100 à 200 °C et en isomérisant ledit au moins un caroténoïde dans de l'huile de triacylglycérol jusqu'à l'obtention d'une teneur en trans-isomère de 40 % à 75 %, et
c) l'introduction de la solution de l'étape b) dans la solution de l'étape a),
d) la réalisation d'au moins une étape de pré-émulsification en faisant passer le mélange de l'étape c) dans un mélangeur à rotor/stator,
e) la réalisation d'au moins une étape d'homogénéisation à haute pression,
f) le séchage par atomisation de l'émulsion de l'étape
e) pour obtenir une poudre,
g-1) la fabrication d'une émulsion en introduisant la poudre de l'étape f) dans de l'eau ; les pourcentages étant des pourcentages pondéraux correspondant à l'émulsion des étapes c) à e),
ou
g-2) la fabrication d'une émulsion en introduisant la poudre de l'étape f) dans de l'eau dans une concentration de 5 % à 70 %, les pourcentages étant des pourcentages pondéraux correspondant à la concentration de la poudre selon l'étape f) dans de l'eau.

6. Procédé selon la revendication 5, comprenant une ou plusieurs des alternatives comprenant :
- l'addition de l'antioxydant hydrosoluble après l'étape c), d) ou e),
- l'addition de l'antioxydant liposoluble à l'étape a), c) ou d) .

7. Poudre comprenant un caroténoïde selon l'étape f) de la revendication 5.

8. Utilisation de la poudre selon la revendication 7 comme colorant, préférablement un colorant naturel ou identique à un colorant naturel, plus préférablement en remplacement de colorants azoïques, dans des aliments, des aliments pour animaux, des préparations pharmaceutiques, et des produits cosmétiques et/ou dermiques.
